# EUROPÄISCHE PATENTSCHRIFT

(11) **EP 1 972 938 B1**
(45) Veröffentlichungstag und Bekanntmachung des Hinweises auf die Patenterteilung: **14.05.2014**
(21) Anmeldenummer: 07104443.2
(22) Anmeldetag: 19.03.2007
(51) Int. Cl.: G01N 33/558, G01N 33/569

(54) **Teststreifen für die Bestimmung des Kariesrisikos**
Test strip for determining the risk of caries
Bande de test pour la détermination du risque de carie

(43) Veröffentlichungstag der Anmeldung: 24.09.2008
(73) Patentinhaber: Ivoclar Vivadent, 9494 Schaan (LI)
(72) Erfinder: Huwig, Alexander Karl, 9470 Buchs (CH); Weigand, Cornelia, 88142 Wasserburg (DE); Goerge, Maja, 9470 Buchs (CH); Grimm, Reto, 7320 Sargans (CH)
(74) Vertreter: UEXKÜLL & STOLBERG

(56) Entgegenhaltungen:
- EP-A- 1 669 758
- EP-A1- 1 396 726
- WO-A-00/11037
- WO-A-94/06940
- WO-A-99/18436
- WO-A-03/023352
- WO-A-2004/034979
- JP-A- 9 292 396
- US-A1- 2003 124 635
- US-A1- 2005 175 992
- US-A1- 2006 166 374
- MEURMAN ET AL: "Five-year follow-up study of saliva, mutans streptococci, lactobacilli and yeast counts in lymphoma patients" EUROPEAN JOURNAL OF CANCER. PART B, ORAL ONCOLOGY, PERGAMON, OXFORD, GB, Bd. 33, Nr. 6, November 1997 (1997-11), Seiten 439-443, XP005021127 ISSN: 0964-1955

## Beschreibung

### GEBIET DER ERFINDUNG

Die Erfindung betrifft ein Teststreifen für die Bestimmung des Kariesrisikos durch semi-quantitativen Nachweis eines kariogenen Mikroorganismus, wie beispielsweise eines bestimmten Bakteriums, in einer Speichel - oder Plaque probe. Schließlich betrifft die Erfindung Kits die den Teststreifen enthalten.

### HINTERGRUND DER ERFINDUNG

Der Nachweis von Mikroorganismen in biologischen Proben, z.B. in Körperflüssigkeiten eines Patienten, ist Ziel zahlreicher im Stand der Technik beschriebener diagnostischer Verfahren. Der erregerspezifische Nachweis kann dabei auf vielfältige Weise erreicht werden. So werden beispielsweise in der human- und veterinärmedizinischen Praxis zahlreiche durch pathogene Mikroorganismen verursachte Infektionen über den Nachweis von erregerspezifischen Nukleinsäuresequenzen (DNA oder RNA) diagnostiziert.

Bei zahlreichen Erkrankungen ist es darüber hinaus hilfreich, eine ungefähre Abschätzung der Konzentration eines bestimmten Mikroorganismen in einer Probe des Patienten vornehmen zu können, beispielsweise um den weiteren Verlauf der Erkrankung prognostizieren zu können. In anderen Fällen erlaubt die Bestimmung der Konzentration von bestimmten Mikroorganismen in einer vom Patienten gewonnenen Probe die Abschätzung des Risikos der Testperson, eine Erkrankung zu entwickeln. So ist beispielsweise das Risiko, Karies zu entwickeln, nachweislich mit einem verstärkten Auftreten bestimmter Keime der Mundflora assoziiert (z.B. Bakterien der Gattung Streptococcus und Lactobacillus). Aus diesem Grund sind Testverfahren entwickelt worden, die eine Überprüfung von Speichelproben im Hinblick auf die Konzentration dieser Keime durch Ausplattieren der Proben auf selektiven Nährböden vorsehen. Die Kultivierung von Proben auf Nährböden ist allerdings verhältnismäßig zeitaufwendig und erfordert ein Anzüchten von Streptococcus und Lactobacillus sowie ein Auszählen von Kolonien dieser Organismen. Eine Auswertung der Ergebnisse kann typischerweise erst nach etwa ein bis drei Tagen oder später nach dem Ausplattieren der Zellen erfolgen.

Alternative Verfahren zur quantitativen oder semi-quantitativen Bestimmung von bestimmten Mikroorganismen in einer Probe basieren auf der Amplifikation von Sequenzen, die für den jeweils nachzuweisenden Mikroorganismus spezifisch sind (z.B. quantitative PCR oder RT-PCR). In diesen Verfahren wird üblicherweise ausgehend von einer Patientenprobe (beispielsweise Blut, Serum, Gewebe, o.ä.) zunächst eine DNA-Aufreinigung durchgeführt. Die aufgereinigte DNA kann anschließend in das jeweilige Amplifikationsverfahren eingesetzt werden, wobei Primer verwendet werden, die hinsichtlich ihrer Sequenz für den nachzuweisenden Erreger spezifisch sind. Die Quantifizierung wird üblicherweise am Ende des Verfahrens durchgeführt, wobei die Menge an PCR Produkt Aufschluß über die ursprünglich in der Probe vorhandene Menge an DNA gibt. Dies kann wiederum mit der Anzahl der ursprünglich in der Probe vorhandenen Zellen korreliert werden.

Die auf der Amplifikation von Nukleinsäuren basierenden Verfahren zum Nachweis von Mikroorganismen weisen allerdings den Nachteil auf, dass verhältnismäßig aufwendige Apparaturen, wie beispielsweise PCR-Thermocycler, zu ihrer Ausführung erforderlich sind. Des Weiteren sind in der Regel weitere Verfahren notwendig, um die Ergebnisse der PCR-Verfahren auszuwerten, z.B. elektrophoretische Trennverfahren. Dies bedingt, dass Schlußfolgerungen bezüglich des Vorliegens eines Mikroorganismus in der untersuchten Probe erst mit einer erheblichen zeitlichen Verzögerung erhalten werden.

In US 2003/0124635 wird ein herkömmlicher immunchromatographischer Teststreifen zur Bestimmung des Kariesrisikos durch Nachweis von Streptococcus mutans beschrieben.

Aufgabe der vorliegenden Erfindung ist es daher, Mittel zur Verfügung zu stellen, mit denen man verläßlich und reproduzierbar das Vorhandensein kariogener Mikroorganismen in einer flüssigen Probe nachweisen und hierbei das Kariesrisiko bestimmen kann. Die beschriebenen Verfahren sollten ohne aufwendige apparative Ausstattung durchführbar sein, und das Ergebnis sollte möglichst innerhalb von 24 Stunden, vorzugsweise von 6 Stunden und besonders bevorzugt innerhalb von 1 Stunde verfügbar sein. Die Mittel sollten darüber hinaus eine semi-quantitative Beurteilung einer untersuchten Probe ermöglichen. Erfindungsgemäß wird die obige Aufgabe durch den Gegenstand der vorliegenden Patentansprüche gelöst.

Die vorliegende Erfindung stellt in einem ersten Aspekt einen Teststreifen für die Bestimmung des Kariesrisikos durch semi-quantitativen Nachweis eines kariogenen Mikroorganismus ausgewählt aus der Gruppe der Mutans-Streptokokken und Lactobacilli in einer flüssigen Speichel- oder Plaque-Probe bereit, wobei der Teststreifen folgendes umfaßt:
a) einen ersten Bereich zur Aufnahme der flüssigen Speichel- oder Plaque-Probe, wobei der erste Bereich einen markierten Antikörper aufweist, der mit dem nachzuweisenden Mikroorganismus unter Ausbildung eines Immunkomplexes reagieren kann;
b) einen zweiten Bereich, der von dem ersten Bereich beabstandet ist und einen Capture-Antikörper aufweist, der mit dem nachzuweisenden Mikroorganismus unter Ausbildung eines Immunkomplexes reagieren kann, und auf dem Teststreifen immobilisiert ist;
wobei der Teststreifen derart gestaltet ist, dass sich der markierte Antikörper bei Kontakt des ersten Bereichs des Teststreifens mit einem Laufmittel in den zweiten Bereich des Teststreifens bewegt, sodass bei Vorliegen des nachzuweisenden Mikroorganismus in der flüssigen Speichel - oder Plaque-Probe, die im ersten Bereich aufgetragen wird, ein Immunkomplex aus dem markierten Antikörper, dem Capture-Antikörper und dem Mikroorganismus gebildet wird, der anhand der Markierung des markierten Antikörpers in dem zweiten Bereich des Teststreifens nachgewiesen werden kann wobei die Stärke des von der Markierung bereitgestellten Signals Aufschluß über die Konzentration des Mikroorganismus in der flüssigen Speichel - oder Plaque-Probe gibt und wobei der Teststreifen ferner eine Kontrolllinie umfasst, deren Intensität auf eine Konzentration von 10⁵ CFU des Mikroorganismus pro ml Speichelprobe eingestellt ist, was bei einem Erwachsenen einem mittleren Risiko entspricht, an Karies zu erkranken, sodass ein stärkeres Signal als die Kontrolllinie auf ein erhöhtes Risiko und ein schwächeres Signal als die Kontrolllinie auf ein niedriges Risiko verweist..

Wie vorliegend verwendet bezeichnet der Begriff "Teststreifen" eine länglich ausgebildete Matrix oder Membran, die vorzugsweise aus einem Material besteht, das einen durch Kapillarkräfte vermittelten lateralen Fluss einer Flüssigkeit oder eines Laufmittels durch die Matrix oder Membran erlaubt. Geeignete Materialien, die eine derartige chromatographische Trennung verschiedener Substanzen in einer flüssigen Probe erlauben umfassen z.B. Cellulose oder Nitrocellulose, Glasfiber, Nylon, Papier, Baumwolle oder teststreifenähnliche Materialien aus Glas, Silicon, Plastik, Metall, in die geeignete Kanäle eingebracht wurden. Geeignete Teststreifen werden von verschiedenen Herstellern in unterschiedlichen Ausgestaltungen angeboten (z.B. die Prima 40-, 60- oder 80-Membranen von Schleicher und Schuell).

Der erfindungsgemäße Teststreifen umfaßt einen markierten Antikörper, der sich (allein oder in Form eines Antigen-Antikörper-Komplexes mit dem nachzuweisenden Mikroorganismus) in einem Flüssigkeitsstrom im Wesentlichen ungehindert durch die Poren des Teststreifens bewegen kann. Darüber hinaus umfaßt der Teststreifen einen in Flussrichtung angrenzenden Bereich mit einem immobilisierten Capture-Antikörper (d.h. ein Antikörper, der so auf dem Teststreifen fixiert ist, dass bei Kontakt mit einem Laufmittel keine Bewegung des Antikörpers durch den Teststreifen möglich ist). Als Laufmittel wird vorliegend eine Flüssigkeit bezeichnet, die den markierten Antikörper aus dem ersten Bereich des Teststreifens in den zweiten Bereich des Teststreifens transportieren kann, wenn sie an dem Ende des Teststreifens, das dem ersten Bereich zugewandt ist, mit dem Teststreifen in Kontakt gebracht wird. Vorzugsweise wird der Teststreifen mit diesem Ende in das Laufmittel eingetaucht. Als Laufmittel dienen herkömmliche Puffer, die nicht mit der Antikörperreaktion interferieren. Auch die Art der Markierung des in der Membran beweglichen Antikörpers spielt für die Zusammensetzung des Laufmittels eine Rolle. Sofern beispielsweise der Antikörper mit einem Enzym markiert ist, dessen Aktivität nachgewiesen werden soll, darf das Laufmittel mit dieser Enzymaktivität nicht interferieren. Geeignete Laufmittel umfassen insbesondere gepufferte Salzlösungen und ähnliches. Darüber hinaus kann das Laufmittel auch Proteinstabilisierende Reagenzien, wie z.B. Serumalbumin, Kohlenhydrate, wie Saccharose, oder Polyole, wie z.B. Sorbit enthalten. Auch Konservierungsmittel, wie z.B. Natriumazid, Benzalkoniumchlorid oder ProClin können zugegeben werden. Sowohl der markierte Antikörper als auch der Capture-Antikörper richten sich gegen einen nachzuweisenden kariogenen Mikroorganismus ausgewählt aus der Gruppe der Mutans-Streptokokken und Lactobacilli.

Bakterien der Gattung Streptococcus und Lactobacillus kommt im Hinblick auf ihre Relevanz für die Bildung von Karies besondere Bedeutung zu (siehe nachstehend). Insbesondere bevorzugt ist es, dass sich die Antikörper gegen Stämme von Streptococcus mutans oder Streptococcus sobrinus, richten. Von diesen Vertretern der Gattung Streptococcus ist bekannt, dass sie als Erstbesiedler der Zahnoberfläche eine Rolle bei der Entstehung von Karies spielen. Alternativ handelt es sich bei dem Mikroorganismus, der mit den erfindungsgemäßen Testsystemen nachgewiesen werden soll, um Stämme von Lactobacillus acidophilus, Lactobacillus gasseri, Lactobacillus casei oder Lactobacillus rhamnosus (Caufield et al. (2007), Caries Res. 41:2-8). Bakterien der Gattung Lactobacillus sind zur fermentativen Umwandlung von Zucker zu Säure fähig und können so die Demineralisierung des Zahnschmelzes fördern.

Der erfindungsgemäß offenbarte Teststreifen, sowie die beschriebenen Nachweisverfahren und Testsysteme, die von diesem Gebrauch machen, eignen sich zur Verwendung bei der Untersuchung von Speichel auf kariogene (d.h. Karies verursachende oder begünstigende) Keime im Mundraum eines Säugetiers, vorzugsweise eines Menschen. Der Mundraum des Menschen ist normalerweise von mehr als 800 unterschiedlichen Keimen besiedelt, die in einem natürlichen Gleichgewicht stehen. Diese Mikroflora, die im Wesentlichen aus Bakterien besteht, umfaßt jedoch auch Organismen, die der Gesundheit der Zähne schaden können. Dies betrifft insbesondere Bakterien, die in der Lage sind, Kohlenhydrate aus der Nahrung zu organischen Säure zu metabolisieren. Die Säure, z.B. Milchsäure, greift den Zahnschmelz an, was zunächst zu einer Demineralisierung der Zahnoberfläche führt. Durch kontinuierliche Besiedelung der demineralisierten Stellen entstehen somit im Laufe der Zeit Läsionen, die durch den Zahnschmelz auf das Zahnbein und das Zahnmark übergreifen können.

Bei der Entstehung von Karies kommt den Bakterien der Gattung Streptococcus eine besondere Bedeutung zu. Insbesondere die Spezies Streptococcus mutans und Streptococcus sobrinus zählen zu den ersten Mikroorganismen, die eine gereinigte Zahnoberfläche neu besiedeln. Beide Spezies sind in der Lage, aus Nahrungsresten und Speichelkomponenten einen polymeren Schleim zu bilden, mit dem sie an der Zahnoberfläche anhaften können. Der von den Streptokokken gebildete Biofilm wird auch als Plaque bezeichnet. Es handelt sich um einen strukturierten Belag aus lebenden und toten Mikroorganismen in einer Matrix aus Polysacchariden und Glycoproteinen. Besonders leicht lagert sich Plaque in den Vertiefungen der Kauflächen (Fissuren), im Bereich zwischen den Zähnen (Interdentalraum) und am Zahnfleischrand an. Der Biofilm ermöglicht auch anderen Bakterien, sich auf der Oberfläche des Zahns niederzulassen. Hierzu zählen insbesondere Bakterien der Gattung Lactobacillus, wie beispielsweise Lactobacillus acidophilus. Bakterien der Gattung Lactobacillus sind zur gemischten Säuregärung fähig und sind für einen großen Teil der gesamten im Mund durch Mikroorganismen gebildeten Säure verantwortlich. An einer aktiven Kariesstelle lassen sich immer Stämme der Gattung Lactobacillus identifizieren. Aus den oben genannten Gründen steht der Nachweis von Bakterien der Gattung Streptococcus und Lactobacillus im Speichel und in der Plaque gegenwärtig im Zentrum der präventionsorientierten Praxis und Forschung.

Beschrieben wird ein Verfahren zur Bestimmung des Kariesrisikos, bei dem die Anzahl der Zellen von Streptococcus mutans (oder S. sobrinus) und mindestens einem Bakterium der Gattung Lactobacillus (im folgenden Lactobacillus sp.) quantitativ oder semi-quantitativ erfaßt wird, wobei die Überschreitung eines definierten Grenzwerts auf ein erhöhtes Risiko für eine Karieserkrankung verweist. Bei Erwachsenen verweist beispielsweise eine Anzahl von 10⁶ Kolonien bildende Einheiten (cfu) pro ml der sogenannten "Mutans-Streptokokken" (unter diesem Begriff werden Streptococcus mutans und Streptococcus sobrinus zusammengefasst) und Lactobacilli auf ein erhöhtes Kariesrisiko. Bei einer Keimzahl von mehr als 10⁶ cfu/ml besteht ein erheblich erhöhtes Risikopotential. Für Kinder gelten etwa 10-fach niedrigere Werte. Dies bedeutet, dass die Verfahren vorzugsweise eine Empfindlichkeit aufweisen sollten, die einen Nachweis von 1000 Zellen/ml gestattet. Dies entspricht bei einem Probevolumen von 10 bis 100 µl einer Genauigkeit von 10 bis 100 Zellen. Eine solche Empfindlichkeit ist beispielsweise bei der Markierung des Antikörpers mit kolloidalem Gold oder kolloidalem Silber gegeben.

Antikörper für den Nachweis von pathogenen Mikroorganismen sind im Stand der Technik hinreichend beschrieben und werden von zahlreichen kommerziellen Anbietern vertrieben. Darüber hinaus können Antikörper gegen einen bestimmten Mikroorganismus mittels bekannter Verfahren hergestellt werden. Die Erzeugung von geeigneten Antikörpern gegen die Mikroorganismen kann ausgehend von molekularen Strukturen, wie beispielsweise Proteinen oder Kohlenhydraten erfolgen, die für den jeweils nachzuweisenden Organismus ausreichend spezifisch sind. Dies bedeutet, dass die verwendeten Antikörper den nachzuweisenden Mikroorganismus anhand bestimmter molekularer Strukturen erkennen und an diese binden kann. Grundsätzlich kann jedes Protein oder Kohlenhydrat, das den nachzuweisenden Mikroorganismus hinreichend spezifiziert, verwendet werden, um geeignete monoklonale oder polyklonale Antikörper gegen Epitope des Proteins unter Verwendung der oben beschriebenen Verfahren zu erzeugen. Besonders bevorzugt handelt es sich bei den als Antigenen fungierenden Molekülen um Proteine des Mikroorganismus, z.B. Zellwandproteine. Diese können durch Anzüchten und Zellaufschluss des entsprechenden Mikroorganismus erhalten werden. Die Zellen werden nach herkömmlichen Verfahren vom Kulturmedium getrennt, in gepufferter Lösung gewaschen (z.B. in phosphatgepufferter Kochsalzlösung) und aufgeschlossen. Für den Zellaufschluss können beliebige der im Stand der Technik bekannten Verfahren eingesetzt werden (Pingoud und Urbanke, 1997, Arbeitsmethoden der Biochemie. Walter de Gruyter-Verlag, Berlin, S. 45-52). Anschließend werden die gewünschten Zellwandbestandteile durch weitere Reinigungsverfahren, z.B. Dialyse, Chromatographie, Filtration, usw., aufgereinigt und gegebenenfalls für eine längerfristige Lagerung lyophilisiert.

Wie beschrieben handelt es sich bei dem Molekül, das für die Erzeugung von Antikörpern verwendet wird, um ein natürlich in dem Mikroorganismus vorkommendes Protein vollständiger Länge oder um eine Variante oder ein Fragment eines solchen Proteins. Unter Varianten eines Proteins oder Polypeptids werden solche Proteine oder Polypeptide verstanden, die sich durch einen oder mehrere Austausche von Aminosäuren von der Aminosäuresequenz des ursprünglichen Proteins oder Polypeptids unterscheiden. Bei dem Aminosäureaustausch kann es sich um einen konservativen oder nicht-konservativen Aminosäureaustausch handeln. Grundsätzlich kann jeder Aminosäurerest der Aminosäuresequenz des ursprünglichen Proteins oder Polypeptids gegen eine andere Aminosäure ausgetauscht sein, sofern der Austausch nicht zu einer wesentlichen Änderung der Struktur des Epitops oder der Epitope des Proteins oder Polypeptids führt. Im Allgemeinen werden Varianten eines Proteins oder Polypeptids eine signifikante Übereinstimmung mit dem ursprünglichen Protein oder Polypeptid aufweisen. Vorzugsweise beträgt die Aminosäureidentität mehr als 60%, 70%, 80%, 90% oder mehr als 95%.

Als Varianten gelten ferner auch solche Proteine oder Polypeptide, die sich von dem ursprünglichen Protein oder Polypeptid durch ein oder mehrere zusätzliche Aminosäuren unterscheiden. Diese zusätzlichen Aminosäuren können sich innerhalb der Aminosäuresequenz des ursprünglichen Proteins oder Polypeptids befinden (Insertion), oder sie können an einen oder an beide Termini des Proteins oder Polypeptids angehängt sein. Insertionen können prinzipiell an jeder Position erfolgen, sofern der Austausch nicht einer wesentlichen Änderung der Struktur des Epitops oder der Epitope des Proteins oder Polypeptids führt. Besonders bevorzugt sind Varianten, bei denen die eine Aminosäure oder mehrere zusätzliche Aminosäuren am C-Terminus und/oder N-Terminus angehängt sind. Somit umfaßt der Begriff Varianten auch Fusionspolypeptide, bei denen das ursprüngliche Protein oder Polypeptid mit flankierenden Sequenzen fusioniert ist, die eine Aufreinigung des Proteins bei heterologer Expression erlauben. Beispiele für solche Sequenzen umfassen Histidinmodule, wie beispielsweise den 6 x His-tag, die über die Affinität zu immobilisierten Nickel-Ionen eine Aufreinigung des Fusionspolypeptids erlauben, oder Domänen des Proteins A, einem bakteriellen Zellwandprotein aus *Staphylococcus aureus* mit einer spezifischen Aktivität zur Fc-Region von Immunglobulinen der G-Klasse (IgG). Andere flankierende Sequenzen, die zur Aufreinigung von Fusionspolypeptiden verwendet werden können sind dem Fachmann hinlänglich bekannt. Ferner umfaßt der Begriff Varianten auch solche Proteine oder Polypeptide, bei denen im Vergleich zu dem ursprünglichen Protein oder Polypeptid eine oder mehrere Aminosäuren fehlen. Solche Deletionen können jede Aminosäure-Position betreffen, sofern der Austausch nicht zu einer wesentlichen Änderung der Struktur des Epitops oder der Epitope des Proteins oder Polypeptids führt.

Beschrieben ist die Verwendung von immunologisch aktiven Fragmenten des ursprünglichen, vom Mikroorganismus erhaltenden Proteins oder Polypeptids und seiner wie oben definierten Varianten zur Herstellung der verwendeten Antikörper. Als Fragmente gelten solche Peptide oder Polypeptide, die sich von dem ursprünglichen Protein oder Polypeptid und seinen Varianten durch das Fehlen einer oder mehrerer Aminosäuren am N-Terminus und/oder am C-Terminus des Peptids oder Polypeptids unterscheiden, wobei zumindest ein Teil der immunologischen Aktivität, d.h. seiner antigenen Eigenschaften, erhalten bleibt.

Derivate des ursprünglichen vom Mikroorganismus erhaltenden Proteins oder Polypeptids oder seiner Varianten können ebenfalls verwendet werden, um die benötigten Antikörper herzustellen. Derivate bezeichnen vorliegend Proteine oder Polypeptide, die gegenüber dem ursprünglichen Protein oder Polypeptid oder seiner Varianten strukturelle Modifikationen, wie beispielsweise modifizierte Aminosäuren, aufweisen. Bei diesen modifizierten Aminosäuren kann es sich erfindungsgemäß um Aminosäuren handeln, die entweder durch natürliche Prozesse, wie beispielsweise Prozessierung oder posttranslationale Modifikationen, oder durch im Stand der Technik hinreichend bekannte chemische Modifikationsverfahren verändert wurden. Typische Modifikationen, denen die Aminosäuren des Polypeptids unterworfen werden können, umfassen Phosphorylierung, Glykosylierung, Acetylierung, Acylierung, Verzweigung, ADP-Ribosylierung, Quervernetzung, Disulfidbrückenbildung, Formylierung, Hydroxylierung, Carboxylierung, Methylierung, Demethylierung, Amidierung, Zyklisierung und/oder kovalente oder nicht-kovalente Bindung an Phosphotidylinositol, Flavin-Derivate, Lipoteichonsäuren, Fettsäuren oder Lipide. Solche Modifikationen sind in der einschlägigen Literatur vielfach beschrieben, z.B. in Proteins: Structure and Molecular Properties, T. Creighton, 2. Auflage, W. H. Freeman and Company, New York (1993).

Der erfindungsgemäße bereitgestellte Teststreifen umfaßt einen ersten Bereich, der zur Aufnahme der flüssigen Probe dient (vorliegend auch als Probenaufnahmebereich bezeichnet). In diesem Bereich enthält der Teststreifen einen markierten Antikörper, der ein nachweisbares Signal bereitstellt. Der Antikörper wird so auf die Matrix aufgebracht, dass er sich bei Kontakt des Teststreifens mit einem Laufmittel in die Flussrichtung, welche von den Kapillarkräften vorgegeben wird, durch die Membran in Richtung der sich anschließenden Nachweiszone wandert. Dies bedeutet, dass der markierte Antikörper nur auf den Teststreifen aufgetragen werden darf, sodass er nur reversibel auf dem Teststreifen gehalten wird und durch den Laufpuffer oder das Probenvolumen mobilisiert werden kann. Sobald der markierte Antikörper in Fluidkontakt mit dem Laufmittel gerät, löst er sich von der Matrix und wird von dem Laufmittel durch die Matrix transportiert. Sofern in der flüssigen Probe, die auf den Probenaufnahmebereich des Teststreifens aufgetragen wurde, der nachzuweisende Mikroorganismus enthalten ist, wird der frei bewegliche markierte Antikörper mit diesem Mikroorganismus reagieren. Dies bedeutet, dass der markierte Antikörper eine antigene Struktur des Mikroorganismus erkennt und an diese bindet (z.B. an ein Oberflächenprotein eines Bakteriums). In diesem Fall wird ein Antigen-Antikörper-Komplex zwischen dem markierten Antikörper und der antigenen Struktur gebildet und durch die poröse Struktur des Teststreifens transportiert. Der Antikörper bzw. die Immunkomplexe bewegen sich anschließend zu einem zweiten Bereich des Teststreifens, der dem ersten Bereich in Flussrichtung nachgelagert ist. In diesem zweiten Bereich (vorliegend auch als Nachweiszone bezeichnet) umfaßt der Teststreifen einen immobilisierten Capture-Antikörper, der sich ebenfalls gegen den nachzuweisenden Mikroorganismus richtet. Dies bedeutet, dass der zweite Antikörper (Capture-Antikörper) sich nicht frei durch den Teststreifen bewegen kann, sondern zum "Einfangen" des vom Mikroorganismus stammenden Antigens dient. Sofern der markierte Antikörper keinen Mikroorganismus gebunden hat (weil der nachzuweisende Mikroorganismus nicht oder nur geringfügig in der Probe vorhanden ist), kann der markierte Antikörper die Nachweiszone ungehindert passieren. Immunkomplexe aus markiertem Antikörper und Mikroorganismus hingegen werden von dem immobilisierten Antikörpern, die eine Spezifität für den Mikroorganismus aufweisen, erkannt und gebunden, sodass eine Art "Sandwich-Komplex" aus den beiden Antikörpern und dem Mikroorganismus gebildet wird. Die Bildung eines solchen Komplexes führt zu einer Anreicherung des markierten ersten Antikörpers in der vom Capture-Antikörper gebildeten Nachweiszone. Hier erfolgt die Auswertung des Tests durch einen geeigneten Nachweis der Markierung des ersten Antikörpers. Die Stärke bzw. Intensität des von der Markierung bereitgestellten Signals gibt dabei Aufschluss über die Menge des auf den Teststreifen befindlichen Mikroorganismus. Es ist bevorzugt, den Capture-Antikörper in Form einer dünnen Linie auf den Teststreifen aufzutragen, sodass die "Sandwich-Komplexe" eine scharfe Bande auf dem Teststreifen bilden. Solche Banden vereinfachen das Auslesen der Signalstärke.

Die erfindungsgemäßen Teststreifen umfassen darüber hinaus einen Kontrollbereich, der sich in Flussrichtung des Laufmittels an die Nachweiszone anschließt und Aufschluß darüber gibt, ob der jeweilige Teststreifen prinzipiell funktionstüchtig ist. Der Kontrollbereich des Teststreifen kann beispielsweise einen immobilisierten Kontrollantikörper umfassen, der einen im Probenaufnahmebereich aufgetragenen mobilen markierten Kontrollantikörper bindet, wobei der mobile markierte Kontrollantikörper weder mit dem immobilisierten Capture-Antikörper in der Nachweiszone noch mit dem nachzuweisenden Mikroorganismus reagiert. Vorzugsweise ist der markierte Kontrollantikörper in der selben Weise markiert wie der markierte Antikörper, der gegen den Mikroorganismus gerichtet ist. Eine weitere Ausführungsform sieht vor, als immobilisierten Kontrollantikörper einen Antikörper zu verwenden, der den markierten, gegen den nachzuweisenden Mikroorganismus gerichteten Antikörper bindet. Dieser immobilisierte Kontrollantikörper kann beispielsweise ein Anti-Spezies-Antikörper sein. Wird z.B. ein monoklonaler Antikörper aus der Maus (wie SWLA1) als markierter Antikörper für die Bindung an den nachzuweisenden Mikroorganismus verwendet, so kann der immobilisierte Kontroll-antikörper ein herkömmlicher Anti-Maus-Antikörper sein. Erst wenn in dem Kontrollbereich ein positives Signal für den zweiten markierten Antikörper erhalten wird, kann das Fehlen eines Signals in der Nachweiszone des Teststreifens dahingehend gedeutet werden, dass der nachzuweisende Mikroorganismus in der Probe nicht enthalten ist oder in einer Konzentration enthalten ist, die unterhalb der Nachweisgrenze liegt.

Eine Verbesserung der Testempfindlichkeit kann durch ein Dotieren der Teststreifen erreicht werden. Dabei werden so viele Zellen des nachzuweisenden Mikroorganismus in den Probenaufnahmebereich aufgebracht, dass eine Konzentration an Zellen unterhalb der Nachweisgrenze erhalten wird. Liegt beispielsweise die Nachweisgrenze für einen bestimmten Mikroorganismus bei 10³ Zellen pro Milliliter, so können dem Probenaufnahmebereich des Teststreifens von 100 Zellen pro Milliliter (10% der Nachweisgrenze) bis zu 900 Zellen pro Milliliter (90% der Nachweisgrenze) zugegeben werden. Bei einer Zugabe von 90% der Nachweisgrenze wird die Empfindlichkeit für den jeweiligen Mikroorganismus auf 10² Zellen pro Milliliter verbessert, da der Teststreifen dann bereits 90 % für einen Nachweis erforderlichen Zellen umfaßt. Für die Dotierung können selbstverständlich auch Teile der nachzuweisenden Mikroorganismen verwendet werden, sofern diese von den entsprechenden Antikörpern des Teststreifen erkannt werden. Dabei kann es sich beispielsweise um antigene Proteine oder Fragmente derselben handeln. Es können auch kurze Peptide verwendet werden, die das Epitop für den zum Nachweis verwendeten Antikörper umfassen. Verwendet werden können Teststreifen, die mit Zellen von Streptococcus mutans oder Streptococcus sobrinus, dotiert wurden. Die Testreifen umfassen vorzugsweise 70%, 80%, 90%, oder 95% der für einen Nachweis erforderlichen Zellen. Die konkrete Nachweisgrenze hängt von der Ausgestaltung des Testsystems und insbesondere von der Markierung des Antikörpers ab und läßt sich für jedes System durch entsprechende Versuche etablieren. Beispielsweise ist der erste Bereich des Teststreifens mit dem nachzuweisenden Mikroorganismus oder mit immunologisch aktiven Teilen desselben dotiert. Vorzugsweise umfaßt der erste Bereich 90% der für den Nachweis erforderlichen Menge des nachzuweisenden Mikroorganismus.

Das Auftragen der Antikörper auf die Teststreifen kann mittels herkömmlicher Dispensierverfahren erfolgen. Dabei wird die jeweilige Antikörperlösung mit einer Kanüle auf die Vliese bzw. Membranen aufgetragen. Die Breite der Testlinie kann hierbei durch die Wahl des Kanülendurchmessers beeinflusst werden. Das Dispensieren erfolgt in der Art, dass die Kanüle über die Vliese bzw. Membranen hinweg bewegt wird. Die jeweils aufgetragene Menge kann eingestellt werden durch die Geschwindigkeit, mit der sich die Kanüle bewegt, sowie durch die Konzentration der jeweiligen Reaktionspartner in der Dispensierlösung. Typische Auftragungsraten liegen für die konjugierten, markierten Antikörper im Bereich zwischen 10-250 optischer Einheiten der gefärbten Kolloide pro 5 mm Vliesbreite. Für die Capture- und Kontrolllinien-Antikörper ergeben sich Raten zwischen 0.01 und 0.5 µl/mm. In diesem Zusammenhang wird auf das IEMA-analogen Testprinzip (immunenzymometrisch-analoges Testprinzip) verwiesen, das beispielsweise in EP-A-0 407 904, EP-A-0 353 570 oder DE-OS 40 24 919 beschrieben ist.

Bei den auf dem erfindungsgemäßen Teststreifen verwendeten Antikörpern handelt es sich um Antikörper, die sich gegen den nachzuweisenden Mikroorganismus richten. Wie vorliegend verwendet umfaßt der Begriff Antikörper Immunglobuline (Ig) sowie immunologisch aktive Teile derselben. Bei den im Rahmen der Erfindung zum Nachweis der Mikroorganismen verwendeten Antikörper kann es sich um beliebige Arten von Antikörpern handeln, z.B. um polyklonale, monoklonale, chimäre, humane, humanisierte, synthetische oder anti-idiotypische Antikörper. Ferner können auch Fragmente der genannten Antikörper eingesetzt werden, um die jeweiligen Mikroorganismen in der Probe nachzuweisen. Auch Antikörper oder deren Fragmente umfassende Fusionsproteine, welche die Sequenz der Antikörper umfaßt, die an ein weiteres Peptid oder Protein fusioniert ist, können im Rahmen der Nachweisverfahren verwendet werden.

Die Antikörper oder deren Fragmente können aus beliebigen Säugetieren erhalten werden, beispielsweise aus Kaninchen, Ratten, Mäusen, Ziegen, Pferden Primaten oder Menschen. Alternativ dazu kann es sich bei den Antikörpern auch um synthetische Antikörper handeln, die beispielsweise mittels rekombinanter Verfahren hergestellt werden. Anhand von Unterschieden in der schweren Kette werden die in Säugetieren gegen Antigene induzierten Antikörper üblicherweise in verschiedene Klassen eingeteilt. Bei den meisten der höher entwickelten Säugetiere gibt es fünf verschiedene Klassen von Immunglobulinen, die als IgG, IgA, IgM, IgD und IgE bezeichnet werden. Daneben können weitere Subklassen existieren. Diese Klassen und Subklassen sind für die Ausführung der offenbarten Verfahren gleichermaßen geeignet. Es ist allerdings bevorzugt, dass es sich bei den Antikörpern um solche der Klasse IgG handelt, z.B. aus den Subklassen IgG1, IgG2, IgG2a, IgG2b oder IgG3.

Wie beschrieben handelt es sich bei den Antikörpern, die zum Nachweis der pathogenen Mikroorganismen eingesetzt werden, um monoklonale Antikörper. Als "monoklonaler Antikörper" wird vorliegend ein Immunglobulin bezeichnet, das von einer Zelllinie produziert wird, die auf einen einzigen B-Lymphozyten zurückgeht. Monoklonale Antikörper richten sich gegen ein einzelnes Epitop in einem bestimmten Antigen. Sie lassen sich durch im Stand der Technik hinreichend beschriebene Verfahren herstellen. Monoklonale Antikörper können beispielsweise unter Verwendung von Hybridom-Zelllinien erzeugt werden, wie es in Köhler und Milstein, Nature, 256, 495-397, (1975) und in Harlow und Lane "Antibodies": Laboratory Manual, Cold Spring, Harbor Laboratory (1988); Ausubel et al., (eds), 1998, Current Protocols in Molecular Biology, John Wiley & Sons, New York) beschrieben wurde. Das Verfahren beruht auf einer Fusionierung einer Antikörper-produzierenden B-Zelle mit einer immortalisierten Zelllinie, wodurch eine hybride Zelle erzeugt wird, die in unbegrenztem Umfang Antikörper einer bestimmten Spezifität produziert. Zunächst wird üblicherweise ein bestimmtes Antigen, gegen das der monoklonale Antikörper erzeugt werden soll, in ein Nagetier injiziert, wie beispielsweise in eine Maus, ein Kaninchen oder eine Ratte. Als Antigen fungiert dabei vorzugsweise ein (gegebenenfalls derivatisiertes) Protein oder Polypeptid oder ein geeignetes Fragment derselben. Durch die Injektion des Antigens wird die Produktion von Antikörpern gegen das Antigen in den B-Lymphozyten des Immunsystems stimuliert.

Die gebildeten Antikörper reichern sich in der Milz und in den Lymphknoten des Tiers an und werden für die Fusion mit Zellen der immortalisierten Zelllinien von dort entnommen. Immortalisierte Zelllinien sind üblicherweise transformierte Zellen aus Säugetieren, insbesondere Myelomzellen von Nagetieren, Rindern oder Menschen. Vorzugsweise werden Myelomzellen aus der Maus verwendet. Gemäß einer alternativen Ausführungsform stammen die Hybridomzellen aus einem Menschen. Ferner sind auch Mensch-Maus-Hetero-Myelomzellen zur Herstellung von humanen monoklonalen Antikörpern beschrieben worden. Die gebildeten Hybridomzellen vereinigen die Eigenschaften ihrer Ursprungszellen. Sie bilden wie der B-Lymphozyt eine bestimmte Antikörper-Spezies und weisen darüber hinaus die Eigenschaft der Myelomzelle auf, sich in vitro unbegrenzt zu vermehren. Die von den Hybridomzellen in das Kulturmedium ausgeschiedenen Antikörpermoleküle können anschließend auf das Vorhandensein des gewünschten monoklonalen Antikörpers untersucht werden.

Die Klone mit den besten Bindungsspezifitäten in Bezug auf das Antigen, beispielsweise das Protein, werden anschließend in einem Screening ermittelt. Die Bindungsspezifitäten der von den Hybridomzellen produzierten Antikörper werden dabei üblicherweise durch Verfahren wie Immunpräzipitation, Radioimmunassay (RIA) oder ELISA (Enzyme Linked Immunosorbent Assay) untersucht. Solche Verfahren sind im Stand der Technik hinreichend beschrieben und bedürfen vorliegend keiner näheren Ausführung. Geeignete Klone werden aufbewahrt, und der Zellüberstand dieser Klone wird bei Bedarf geerntet.

Hybridomzellen können bei einer Kultivierung in Rollerflaschen von 5 1 zu einer Ausbeute von 20-70 mg des gewünschten monoklonalen Antikörpers führen. Darüber hinaus können Hybridomzellen zur Herstellung von Antikörpern im großen Maßstab von 200-600 mg eingesetzt werden, indem Tiere, wie beispielsweise Mäuse, unmittelbar mit diesen Hybridomzellen immunisiert werden, und die im Verlauf der anschließenden Infektion gebildeten Antikörper in der Bauchhöhlenflüssigkeit (Aszites) gesammelt werden (Leenaars und Hendriksen (2005), ILAR J, 46:269-279; Hendriksen und de Leeuw (1998), Res Immunol, 149:535-542).

Neben Verfahren, die auf der Erzeugung von Hybridomzellen beruhen, sind weitere Verfahren zur Herstellung monoklonaler Antikörper beschrieben worden. Diese Verfahren sind gleichermaßen zur Erzeugung der Antikörper geeignet. Beispielsweise können monoklonale Antikörper durch rekombinante DNA-Verfahren hergestellt werden, wie es beispielsweise in US-Patent Nr. 4,816,567 beschrieben ist. Monoklonale Antikörper können darüber hinaus aus einer Antikörper-Phagenbibliothek isoliert werden, wie es z.B. in Clackson et al. (1991), Nature, 352:624-628, beschrieben wurde.

Sofern der erfindungsgemäße Teststreifen für den Nachweis von Streptococcus mutans ausgebildet ist, kann es sich bei dem markierten Antikörper und/oder dem Capture-Antikörper der Membran beispielsweise um den monoklonalen Antikörper SWLA1 (ATCC HB12559), SWLA2 (ATCC HB12560) oder SWLA3 (ATCC HB12558) handeln, die in WO 00/11037 beschrieben sind. Bei dem monoklonalen Antikörper SWLA1, der aufgrund einer hohen Sensitivität gegen Streptococcus mutans selektioniert worden ist, handelt es sich um ein Immunglobulin vom Typ IgG, das für den Test mit kolloidalem Silber oder Gold einer Partikelgröße von 20 bis 300 nm, vorzugsweise 20 bis 80 nm und besonders bevorzugt 40 nm konjugiert wird (siehe auch Shi et al. (1998) Hybridoma, 17:365-371; Gu et al.(2002), Hybrid Hybridomics, 21:225-232). Sofern der erfindungsgemäße Teststreifen für den Nachweis von Bakterien der Gattung Lactobacillus ausgebildet ist, kann es sich bei dem markierten Antikörper und/oder dem Capture-Antikörper der Membran beispielsweise um den monoklonalen Antikörper SWLA5 vom Typ IgM handeln (Gu et al. (2002), Hybrid Hybridomics, 21:469-478). Bei der Detektion der Lactobacilli ist keine Spezifität im Hinblick auf die Art oder den Stamm erforderlich, d.h. alle für Bakterien der Gattung Lactobacillus hinreichend spezifischen Antikörper sind zur Verwendung in den Verfahren und Testsystemen der vorliegenden Erfindung geeignet.

Im Rahmen der Erfindung können im Zusammenhang mit den beschriebenen Verfahren und Testsystemen auch polyklonale Antikörper verwendet werden. Polyklonale Antikörper werden aus Seren von Tieren erhalten, die mit einem Antigen (z.B. einem bestimmten Protein) immunisiert worden sind. Polyklonale Antikörper sind letztlich eine heterogene Mischung von Antikörpern, die von mehr als einem B-Lymphozyten abstammen und sich oftmals gegen verschiedene Epitope eines bestimmten Antigens richten. Wie vorliegend verwendet umfaßt der Begriff "polyklonaler Antikörper" auch einen monospezifischen Antikörper, der nach Aufreinigung der aus den Seren gewonnenen verschiedenen Antikörpern (z.B. mit Hilfe einer Säule, an die Peptide gekoppelt sind, welche ein bestimmtes Epitop enthalten) erhalten wird.

Bei der Herstellung von polyklonalen Antikörpern wird zunächst ein Wirtstier (z.B. ein Kaninchen, eine Ziege, eine Maus oder ein anderes Säugetier) durch eine oder mehrere Injektionen des Antigens, z.B. des immunogenen Proteins (oder eines Fragments oder einer Variante davon), immunisiert. Dies bedeutet, daß das Antigen diesen Tieren ein- oder mehrmals injiziert wird. In die immunogene Zusammensetzung kann ein natürlich vorkommendes, natives Protein, ein chemisch synthetisiertes Polypeptid, das dieses Protein oder einen Teil davon darstellt oder ein rekombinant exprimiertes Protein oder ein Fragment davon eingesetzt werden. Das Protein kann ferner an ein weiteres Protein gekoppelt werden, von dem bekannt ist, daß es in dem als Wirt verwendeten Säugetier immunisierend wirkt. Solche akzessorischen Proteine sind im Stand der Technik hinreichend bekannt und umfassen z.B. das sogenannte Keyhole-Limpet-Hämocyanin, Serumalbumin, Sojabohnen-Trypsininhibitor und andere. Die zur Immunisierung verwendeten Zusammensetzungen umfassen des weiteren in der Regel ein Adjuvanz. Als "Adjuvanz" wird vorliegend eine Substanz bezeichnet, die immunologische Reaktion des Wirts verstärkt. Solche Adjuvanzien sind ebenfalls auf dem Gebiet der Immunologie bekannt und umfassen beispielsweise Freudsches vollständiges oder unvollständiges Adjuvanz sowie Aluminiumhydroxid und andere.

Bei den im Rahmen der beschriebenen Verfahren verwendeten Antikörpern kann es sich auch um chimäre Antikörper handeln. Wie vorliegend verwendet bezeichnet der Begriff "chimärer Antikörper" einen Antikörper der aus verschiedenen Bestandteilen zusammengesetzt ist, die aus verschiedenen Säugetier-Spezies stammen. Chimäre Antikörper sind somit "gemischte" Antikörper, die aus molekularen Bauteilen aus (mindestens) zwei verschiedenen Antikörpern bestehen. Üblicherweise leiten sich die variablen Regionen eines chimären Antikörpers von einer Säugetier-Spezies ab, während die konstanten Regionen von einer anderen Spezies stammen. Beispiele für chimäre Antikörper umfassen beispielsweise humanisierte Antikörper. Chimäre Antikörper und Verfahren zu ihrer Herstellung sind dem auf dem Gebiet tätigen Fachmann hinreichend bekannt und wurden darüber hinaus beispielsweise in Boulianne et al. (1984), Nature 312:643-646; Cabilly et al. (1984), Proc. Natl. Sci. USA 81:3273-3277 und Neuberger et al. (1985), Nature 314:268-270 beschrieben. Prinzipien und Erwägungen, die bei der Konstruktion von chimären Antikörpern relevant sind, können auch in Harlow und Lane "Antibodies": Laboratory Manual, Cold Spring, Harbor Laboratory (1988) gefunden werden.

Darüber hinaus können neben vollständigen Antikörper-Molekülen auch funktionelle Fragmente von Antikörpern für die Verfahren und Testsysteme Anwendung finden. Als funktionelle Fragmente werden vorliegend solche Teile der wie oben definierten Antikörper verstanden, die zumindest einen Teil der Bindungsaffinität des vollständigen Antikörpers, von dem sie abgeleitet sind, beibehalten haben, und somit in der Lage sind, mit einer hinreichenden Spezifität an das entsprechende Antigen (vorliegend an den nachzuweisenden Mikroorganismus) zu binden. Fragmente von Antikörpern werden üblicherweise durch proteolytische Spaltung, chemische Synthese oder rekombinante DNA-Verfahren hergestellt. Beispiele für geeignete Antikörperfragmente umfassen Fab, Fab', F(ab')₂ und Fv-Fragmente sowie einzelsträngige Antikörper mit Bindungsaffiniät für den nachzuweisenden Mikroorganismus. Besonders bevorzugt sind Fragmente der monoklonalen Antikörper SWLA1, SWLA2, SWLA3 oder SWLA5.

Es ist erfindungsgemäß bevorzugt, dass es sich bei dem markierten Antikörper und dem Capture-Antikörper um denselben Antikörper handelt. Dies ist allerdings nicht zwingend notwendig, solange gewährleistet ist, dass sowohl der markierte Antikörper als auch der Capture-Antikörper mit einer hinreichenden Spezifität an den jeweils nachzuweisenden Mikroorganismus, z.B. Streptococcus mutans oder Lactobacillus sp., bindet. Es können beispielsweise Antikörper verwendet werden, die sich gegen verschiedene Moleküle, z.B. verschiedene Zellwandproteine, desselben Mikroorganismus richten. Ferner können beispielsweise Glycerophosphate und Ribitphosphate, die mit weiteren Zuckern sowie mit D-Alanin verknüpft sind, erkannt werden, wobei diese Einheiten wiederum mit Membranlipiden verknüpft sein können. Auch Fette können als antigene Strukturen dienen.

Ferner können auch Antikörper verwendet werden, die sich gegen dasselbe Molekül richten, wobei jedoch unterschiedliche Epitop-Strukturen erkannt werden. Gemäß einer bevorzugten Ausführungsform ist entweder der markierte Antikörper oder der Capture-Antikörper ein monoklonaler Antikörper. Besonders bevorzugt ist es, dass sowohl der markierte Antikörper als auch der Capture-Antikörper monoklonale Antikörper sind.

Gemäß einer besonders bevorzugten Ausführungsform der Erfindung ist der markierte, gegen den nachzuweisenden Mikroorganismus gerichtete Antikörper an Partikel konjugiert, die ein colorimetrisches Signal bereitstellen. Solche Partikel sind im Stand der Technik hinreichend bekannt und umfassen beispielsweise Partikel aus kolloidalem Gold oder kolloidalem Silber. Kolloidales Gold zeichnet sich durch eine tiefrote Farbe aus, die visuell sehr leicht wahrzunehmen ist. Kolloidales Silber hingegen weist eine gelbliche Färbung im verdünnten Zustand und eine braune Färbung im konzentrierten Zustand auf. Die Partikel weisen erfindungsgemäß eine Größe von etwa 10-100 nm auf, wobei Partikelgrößen von etwa 20-80 nm und insbesondere etwa 40 nm besonders bevorzugt sind. Neben kolloidalem Gold oder Silber können die Antikörper auch an gefärbte Latex-Partikel gekoppelt werden. Alternativ dazu können auch andere dem Fachmann geläufige Moleküle zur Markierung der Antikörper verwendet werden. Solche Markierungen umfassen insbesondere fluoreszente Markierungen, chemilumineszente Markierungen, Biotin, Avidin, Streptavidin, chromogene Gruppen, die bei ihrer Hydrolyse einen visuell erkennbaren Farbstoff bilden, oder Enzyme. Bevorzugte Enzyme umfassen Phosphatasen (z.B. alkalische Phosphatase), Peroxidasen (z.B. Meerrettich-Peroxidase), beta-Galactosidase. Darüber hinaus können die Antikörper, die auf den Teststreifen aufgebracht sind, auch mit sekundären Antikörpern nachgewiesen werden, die spezifisch an die Antikörper auf dem Teststreifen binden. Die sekundären Antikörper können zum Zwecke des Nachweises mit entsprechenden Molekülen, wie z.B. den oben genannten Enzymen, markiert sein.

Bei der flüssigen Probe, welche den nachzuweisenden Organismus umfaßt, handelt es sich um eine Speichel- oder Plaque-Probe. Je nach Art und Herkunft der Probe und/oder der Art des nachzuweisenden Mikroorganismus kann es notwendig oder sinnvoll sein, vor Durchführung des erfindungsgemäßen Verfahrens zunächst eine Aufkonzentrierung des Zellmaterials durchzuführen. Eine solche Aufkonzentrierung kann unter Verwendung eines Filters geeigneter Porengröße durchgeführt werden. Die auf dem Filter verbliebenen Zellen können anschließend in einem geringeren Volumen Flüssigkeit resuspendiert werden.

Sofern erforderlich können die als Proben verwendeten Flüssigkeiten mit weiteren Mitteln behandelt werden, um die Zugänglichkeit der nachzuweisenden Mikroorganismen zu erhöhen. Dadurch läßt sich in der Regel die Empfindlichkeit des jeweiligen Testsystems deutlich erhöhen. Zu diesem Zweck wird die zu untersuchende Probe mit Substanzen versetzt, die eine Interaktion der Organismen mit anderen Bestandteilen der Probe oder eine Interaktion der Organismen untereinander verringern. Diese Substanzen umfassen chelatisierende Moleküle, z.B. EDTA, NTA, DTPA, HEDTA oder Citronensäure, die Interaktionen der Mikroorganismen mit Kationen wie Ca²⁺, Cu²⁺, Mg²⁺, Fe²⁺, Fe³⁺ oder Co³⁺ durch Komplexierung der Kationen unterbinden. Ferner können die Proben vor der Aufbringung auf die Teststreifen mit proteolytischen Enzyme vorbehandelt werden. Proteolytisch aktive Enzyme sind dem Fachmann hinreichend bekannt. Sie umfassen beispielsweise die Enzyme Pepsin, Papain, Pancreatin, Trypsin, Chymotrypsin, Chymosin, Renin, Cathepsin B und D und ähnliche. Diese Proteasen bewirken die Spaltung und den Abbau von Proteinen der Probe, z.B. Speichelproteinen (Mucinen), sowie von Proteinen, die eine Aggregationen zwischen einzelnen Zellen des Mikroorganismus bewirken oder unterstützen. Auch zuckerspaltende Enzyme, wie z.B. Glucosidasen, Amylasen, Lysozym, Hyaluronidasen und ähnliche können zur Verbesserung der Testempfindlichkeit beitragen, indem sie Glycoproteine und/oder Kohlenhydratbestandteile der mikrobiellen Zellwände angreifen. Chaotrope Verbindungen und Ionen der Hofmeister-Serie können ebenfalls verwendet werden. Sie verringern das Ausmaß von unspezifischen Wechselwirkungen, z.B. ionische Wechselwirkungen, Wasserstoffbrückenbindungen, Coulomb-Wechselwirkungen. SH-Gruppen-modifizierende Reagenzien, wie z.B. Dithiothreitol, Mercaptoethanol, Glutathion und andere können der Probe zur Spaltung von Proteinunterheiten zugesetzt werden, die durch Disulfidbrücken verbunden sind. Darüber hinaus können auch Lösungen oder Substanzen zu der jeweiligen Probe gegeben werden, die den pH-Wert verändern (z.B. NaOH, HCl oder gepufferte Lösungen); durch die Veränderung des pH-Werts der flüssigen Probe kann eine Änderung ihrer Konsistenz erreicht werden, die für die chromatographische Trennung auf den Teststreifen vorteilhaft ist.

Die Probe kann mittels geeigneter Instrumente auf den Teststreifen überführt werden. Dabei können prinzipiell alle Instrumente, die in reproduzierbarer Weise die Auftragung eines bestimmten Volumens der Probe auf die Teststreifen ermöglichen, verwendet werden. Beispielsweise wird der Probentransfer auf den Teststreifen möglichst einfach, z.B. mit einer Pipette, gestaltet. Ferner sind im Stand der Technik geeignete Pinselähnliche Applikatoren beschrieben, wie sie z.B. von der Firma Microbrush (Grafton, WI, USA) hergestellt werden, die einen gut reproduzierbareren Transfer eines definierten Probenvolumens, wie beispielsweise eines Speichelvolumens ermöglichen. Darüber hinaus bietet ein derartiger Applikator die Möglichkeit, Plaque-Proben gezielt von einzelnen Zähnen zu entnehmen und in einen Puffer, z.B. in den Laufpuffer, zu überführen. Auf diese Weise ist es beispielsweise möglich, das Erkrankungsrisiko an einer bestimmten Stelle zu bestimmen. Ferner sind die Entnahme und der Transfer von Sulkusflüssigkeit bzw. von Probenmaterial aus einer Zahnfleischtasche möglich. Aus Gründen der Reproduzierbarkeit sollte jede einzelne Bestimmung eines Mikroorganismus mit einem separaten Applikator erfolgen. Das Volumen, das auf die Teststreifen aufgetragen wird, ist von der Art der zu untersuchenden Probe und der zu erwartenden Konzentration des darin enthaltenden, nachzuweisenden Mikroorganismus abhängig. Sofern es sich beispielsweise bei der Probe um eine Speichelprobe handelt, ist das Probenvolumen in der Regel geringer als 500 µl, beispielsweise 300 µl, 100 µl, 50 µl oder 10 µl. Das Probevolumen wird durch die Art des Antikörpers, die Art der Visualisierung der Antigen-Antikörper-Reaktion und durch die Materialien zur Herstellung der Teststreifen beeinflusst und wird in Abhängigkeit von diesen Faktoren immer maximal niedrig eingestellt.

Beschrieben wird ferner Verfahren zum quantitativen oder semi-quantitativen Nachweis eines Mikroorganismus in einer flüssigen Probe bereit, bei dem man
a) die flüssige Probe auf einen wie oben definierten Teststreifen aufträgt,
b) den Teststreifen mit einem Laufmittel in Kontakt bringt,
c) den Teststreifen unter Bedingungen inkubiert, bei denen sich der markierte Antikörper bei Kontakt des ersten Bereichs des Teststreifens mit einem Laufmittel in den zweiten Bereich des Teststreifens bewegt,
d) das Signal in dem zweiten Bereich des Teststreifens detektiert;
wobei die Stärke des von der Markierung bereitgestellten Signals Aufschluss über die Konzentration des Mikroorganismus in der flüssigen Probe gibt.

Das beschriebene Verfahren sieht zunächst vor, dass die zu untersuchende flüssige Probe auf den vorgesehenen Probenaufnahmebereich des Teststreifens aufgetragen wird. Sofern die Probe eine ausreichend niedrige Viskosität und ein ausreichendes Volumen aufweist, ist die Verwendung eines Laufmittels nicht erforderlich. Es ist natürlich auch möglich die zu untersuchende Probe in einem geeigneten Laufmittel einzubringen und ein entsprechend großes Volumen auf den Probenaufnahmebereich des Teststreifen aufgetragen. Bessere Ergebnisse werden allerdings erzielt, indem man ein verhältnismäßig kleines Probenvolumen von unter 500 µl auf den Teststreifen aufträgt und durch Eintauchen des Endes des Teststreifens, das dem Probenaufnahmebereich zugewandt ist, in ein entsprechendes Laufmittel die Chromatographie startet. Anschließend wird der Teststreifen unter Bedingungen inkubiert, die eine Ausbreitung der flüssigen Probe vom ersten Bereich des Teststreifens (d.h. dem Probenaufnahmebereich) in den durch die kapillare Flussrichtung vorgegebenen angrenzenden zweiten Bereich des Teststreifens (d.h. die Nachweiszone) erlauben. Sofern eine Bindung des markierten Antikörpers in der Nachweiszone des Teststreifens erfolgt, kann das von der Markierung bereitgestellte Signal in diesem Bereich detektiert werden. Die Detektion richtet sich nach der Art der Markierung. Beispielsweise handelt es sich bei der Markierung, die an dem ersten Antikörper angebracht ist, um kolloidales Gold oder kolloidales Silber, sodass die Detektion durch visuelle Sichtprüfung erfolgen kann. Je nach Art der Markierung kann es allerdings auch erforderlich sein, eine Enzymreaktion mit einem lumineszenten Substrat durchzuführen, oder eine fluoreszente Markierung nachzuweisen (z.B. mit Hilfe eines Fluoreszenzmikroskops). Die Antigen-Antikörper-Reaktion kann auch durch Licht- bzw. Reflexionsmessung kontinuierlich ausgelesen werden.

Als letzten Schritt umfaßt das beschriebene Verfahren den Vergleich des detektierten Signals mit einem oder mehreren Referenzwerten, um eine quantitative oder semi-quantitative Aussage in Bezug auf den nachzuweisenden Mikroorganismus zu treffen. Vorzugsweise wird dabei ein Farbstandard, der die semi-quantitative Abschätzung der in der Probe vorliegenden Anzahl des nachzuweisenden Mikroorganismus angibt. Um die Anwendung des Tests so einfach wie möglich und unabhängig von zusätzlichen Geräten durchführen zu können, ist es bevorzugt, dass die Signalintensität durch Vergleich mit einem optischen Standard ausgelesen wird, der die Signalintensität stufenweise widerspiegelt. Dies entspricht einer weiteren Vereinfachung, da das Signal diskontinuierlich in Stufen, d.h. semi-quantitativ, bestimmt werden kann. Die Abstufungen des Standards werden dabei anhand von definierten Konzentrationen der nachzuweisenden Mikroorganismen erstellt. Die optischen Standards können insbesondere bei einer Markierung des ersten Antikörpers mit kolloidalem Gold oder kolloidalem Silber verwendet werden. Auch Antikörper, die mit Enzymen markiert sind, erlauben die Erstellung von optischen Standards. Vorzugsweise umfassen die optischen Standards mehrere Stufen, die sich hinsichtlich der Signalintensität, z.B. der Farbtiefe, voneinander unterscheiden. Besonders bevorzugt sind optische Standards mit vier Stufen. Die optischen Standards lassen sich durch Verdünnungsreihen herstellen, bei denen Proben mit bekannter Keimzahl unter Versuchsbedingungen auf die Teststreifen aufgetragen werden und die Signalstärke für jede Probe als Referenzwert für eine bestimmte Anzahl des jeweiligen Mikroorganismus verzeichnet wird. Es können selbstverständlich auch Kalibrierungskurven aufgenommen werden, die eine Zuordnung eines nachgewiesenen Signals zu einer bestimmten Anzahl von Mikroorganismen erlauben.

Die erfindungsgemäße binäre Einstufung erfolgt anhand der Kontrolllinie des Teststreifens. Die Intensität der Kontrolllinie wird auf eine für jeden Mikroorganismus festzulegende Konzentration eingestellt (10⁵ cfu/ml), sodass anhand dieser Kontrolllinie eine zusätzliche binäre Bewertung möglich ist. Bei einem vierstufigen Standard kann beispielsweise die zweithöchste Konzentrationsstufe auf die Kontrolllinie bezogen werden, d.h. wenn das Testsignal hinsichtlich seiner Intensität dem Kontrollliniensignal entspricht, so liegt die zweithöchste Konzentration des Mikroorganismus in der Probe vor. In Abhängigkeit davon ergibt sich folgende Intensitätseinteilung:
Testsignal > Kontrolllinie: höchste Konzentration
Testsignal = Kontrolllinie: zweithöchstes Konzentration
Testsignal < Kontrolllinie: zweitkleinstes Konzentration
Testsignal = 0 oder << Kontrolllinie: geringste Konzentration

Um Fehler zu vermeiden sollte für alle Untersuchungen versucht werden, zumindest ein schwaches Signal zu erhalten, damit die Einschätzung der Konzentration immer auf der Basis eines Signals erfolgt.

Für die Abschätzung des Kariesrisiko entsprechen 10⁵ cfu Mutans-Streptokokken (Streptococcus mutans, Streptococcus sobrinus) oder 10⁵ cfu Lactobacilli pro ml Speichelprobe bei einem Erwachsenen einem mittleren Risiko, an Karies zu erkranken. Auf diesen Wert wird die Kontrolllinie eingestellt, sodass ein stärkeres Signal als die Kontrolllinie ein erhöhtes Risiko und ein schwächeres Signal als die Kontrolllinie ein niedriges Risiko indiziert. Diese Einstellung wird bei der Bewertung der Teststreifen genutzt, um dem Anwender eine Auswertung des Kariesrisikos in hoch oder niedrig zu erlauben. Die genauere Einteilung in eine von mindestens vier Stufen kann dann beispielsweise durch einen Vergleich mit einem Auswertungsbogen als optischen Standard erfolgen. Die untere, durch die Empfindlichkeit vorgegebene Grenze liegt bei 5x10³ cfu/ml. Erscheint auf der Testlinie kein oder nur ein schwaches Signal entspricht dies der Stufe 0. Ist das Signal deutlich vorhanden, aber schwächer als die Kontrolllinie, d.h. z.B. etwa 10⁴, wird dem Signal die Stufe 1 zugeordnet. Stufe 2 ist gegeben, wenn Testlinie und Kontrolllinie gleich stark sind. Stufe 3, d.h. die höchste von 4 Stufen, ist dann gegeben, wenn mehr als 5x10⁵ cfu/ml vorliegen).

Gemäß der vorliegenden Erfindung ist der Teststreifen zum semi-quantitativen Nachweis eines Mikroorganismus in einer flüssigen Probe ein zur Abschätzung des Risikos für eine Karieserkrankung. Gemäß der vorliegenden Erfindung wird bei diesem Kariesrisikotest die Keimzahl von Streptococcus mutans und/oder Lactobacillus sp. semi-quantitativ bestimmt. Besonders bevorzugt ist es, im Rahmen des Nachweisverfahrens parallel mindestens zwei Bakterienarten gleichzeitig in einer Probe, wie beispielsweise in derselben Speichelprobe zu bestimmen. Dazu können zwei Teststreifen gleichzeitig verwendet werden. Der erste Teststreifen umfaßt dabei einen gegen Streptococcus gerichteten Antikörper, z.B. SWLA1 als markierten Antikörper und/oder als Capture-Antikörper. Der zweite des Teststreifens umfaßt einen gegen Lactobacillus gerichteten Antikörper, z.B. SWLA5 als markierten Antikörper und/oder als Capture-Antikörper.

Schließlich betrifft die vorliegende Erfindung auch ein Kit . Das erfindungsgemäße Kit umfaßt eine oder mehrere der oben beschriebenen Teststreifen sowie gegebenenfalls einen oder mehrere Puffer, ein oder mehrere Laufmittel, ein oder mehrere Markierungsreagenzien und/oder ein oder mehrere Detektionsreagenzien zum Nachweis des markierten Antikörpers. Darüber hinaus kann das Kit Anweisungen zur Durchführung umfassen. Üblicherweise kann das Kit auch geeignete Vergleichstandards, die eine Zuordnung von Signalstärke und Konzentration des Mikroorganismus in der Probe ermöglichen, umfassen. Im Falle einer colorimetrischen Markierung des zur Detektion verwendeten markierten Antikörpers (z.B. kolloidales Gold) ist der Vergleichstandard beispielsweise eine Farbtafel, die eine Korrelation von Farbtiefe und Konzentration des Mikroorganismus erlaubt.

Beschrieben wird eine Testvorrichtung für den parallelen, gleichzeitigen Nachweis von zwei Mikroorganismen, beispielsweise Streptococcus mutans und Lactobacillus sp., in einer flüssigen Probe bereitgestellt. Das Testsystem umfaßt zwei der wie oben definierten Teststreifen und ein Gehäuse, das zwei Paare aus jeweils einer ersten Öffnung und einer von der ersten Öffnung beabstandeten zweiten Öffnung und eine Einführöffnung für die zwei Teststreifen aufweist, wobei die Einführöffnung und die zwei Paare aus der ersten Öffnung und der zweiten Öffnung derart angeordnet sind, dass die zwei Teststreifen so durch die Einführöffnung in das Gehäuse eingeführt werden können, dass jeweils zwei voneinander beabstandete Bereiche der Teststreifen durch die ersten Öffnungen und die zweiten Öffnungen zugänglich sind. Dabei ist jeweils der Probenaufnahmebereich des Teststreifens durch die erste Öffnung zugänglich, während die Nachweiszone durch die zweite Öffnung zugänglich ist.

Die Verwendung eines erfindungsgemässen Teststreifens oder eines wie oben beschriebenen Testsystems zur Bestimmung des Risikos für eine Karieserkrankung, ist ebenfalls Gegenstand der vorliegenden Erfindung.

Im Folgenden wird die Zeichnung näher erläutert.

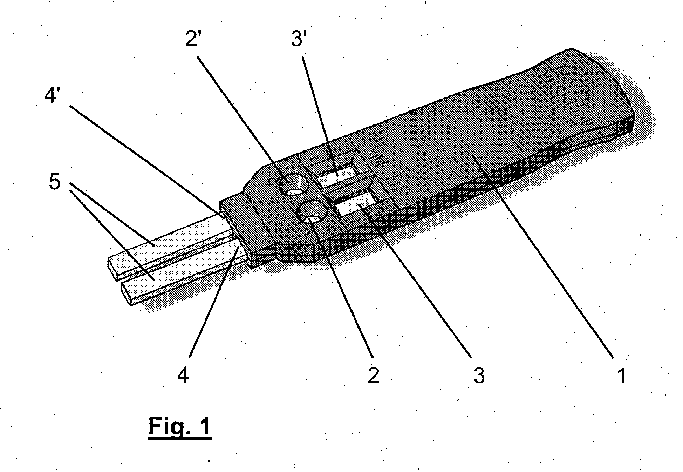

In Fig. 1 ist ein Testsystem dargestellt. Das Testsystem umfaßt ein Gehäuse 1, das eine erste, obere Halbschale und eine zweite untere Halbschale umfaßt. In dem Gehäuse 1 sind in der oberen Halbschale zwei Paare aus jeweils einer ersten Öffnung 2, 2' und einer zweiten Öffnung 3, 3' vorgesehen. Dabei sind die erste Öffnung 2, 2' und die zweite Öffnung 3, 3' der beiden Paare beabstandet voneinander angeordnet, wobei die Verbindungslinien zwischen der ersten Öffnung 2, 2' und der ersten zweiten Öffnung 3, 3' der beiden Paare parallel zueinander verlaufen.

Des Weiteren ist in dem Gehäuse 1 eine Einführöffnung vorgesehen, die einen ersten Öffnungsbereich 4 und einen zweiten Öffnungsbereich 4' umfaßt. Die Einführöffnung 4, 4' ist so in dem Gehäuse 1 angeordnet, dass zwei Teststreifen 5 derart durch die Öffnungsbereiche 4, 4' der Einführöffnung in das Gehäuse 1 eingeführt werden können, dass jeweils zwei voneinander beabstandete Bereiche eines Teststreifens 5 durch die erste Öffnung 2, 2' und die zweite Öffnung 3, 3' eines Paares der Öffnungen zugänglich sind. Wie im Folgenden erläutert, dienen die ersten Öffnungen 2, 2' als Probenöffnung und die zweiten Öffnungen 3, 3' als Fenster zum Ablesen der Antigen-Antikörperreaktion. Dies bedeutet, dass die Bereiche der Teststreifen, die durch die ersten Öffnungen (2, 2') zugänglich sind, den markierten Antikörper aufweisen, während die Bereiche der Teststreifen, die durch die zweiten Öffnungen (3, 3') zugänglich sind, den Capture-Antikörper und ggf. eine oder mehrere Kontrolllinien aufweisen.

Um mit dem Testsystem eine Messung zur Bestimmung des Kariesrisikos bei einem Subjekt durchzuführen, wird zunächst in die Probenöffnungen bzw. die beiden ersten Öffnungen 2, 2' der beiden Öffnungspaare eine Speichelprobe eingefüllt. Da die Speichelprobe in der Regel eine zu hohe Konsistenz aufweist, um die chromatographische Trennung auf dem Teststreifen zu initiieren, werden die beiden aus den Öffnungsbereichen 4, 4' der Einführöffnung herausragenden Teststreifen 5 in ein geeignetes Laufmittel getaucht, das anschließend aufgrund von Kapillarkräften durch die gesamten Teststreifen 5 gezogen wird.

In der Folge wird auch der in die ersten Öffnungen 2, 2' eingefüllte Speichel aus dem Bereich der ersten Öffnungen 2, 2' in den Bereich der Ablesefenster bzw. der zweiten Öffnungen 3, 3' gezogen und führt dort jeweils die oben bereits beschriebenen Reaktionen aus. Anschließend kann der Benutzer aufgrund der Verfärbung des Teststreifens 5 im Bereich der zweiten Öffnungen 3, 3' das Ergebnis der Antikörperreaktion ablesen. Das Testsystem ermöglicht somit in leicht handhabbarer Weise die gleichzeitige Untersuchung einer flüssigen Probe in Bezug auf zwei unterschiedliche Mikroorganismen, z.B. Streptococcus mutans und Lactobacillus sp.

Die nachfolgenden Versuche beschreiben das Testsystem zur Bestimmung von pathogenen Mikroorganismen in der Mundhöhle.

### 1. Untersuchung des Kariesrisikos durch Nachweis von S. mutans und Lactobacillus sp.

### 1.1 Herstellung der Teststreifen

Auf eine selbstklebende Folie werden kapillaraktive, chromatographiefähige, flächenförmige Materialien wie Membranen und Vliese fixiert. Alle Materialien stehen in einem kapillaren Kontakt miteinander und erlauben einen Flüssigkeitsstrom von einem Ende des Teststreifens bis zum anderen. Die Montage der Materialien erfolgt in der Art, dass an der Einführöffnung in die Kassette Saugvliese vorhanden sind, die den Laufpuffer bzw. eine Probe mit einem Volumen von mehr als 50 µl absorbieren und konstant freisetzen. Die Saugvliese stehen in Kontakt mit den Freisetzungsvliesen, auf die je mindestens ein mobilisierbarer Antikörper-Konjugat-Komplex aufgebracht sind. Zur Erhöhung der Testempfindlichkeit kann das Freisetzungsvlies mit Antigen, d.h. mit ganzen Zellen von Mikroorganismen, vorzugsweise aber mit die entsprechenden Epitope enthaltenen Zellfragmenten, dotiert werden. Auf das Freisetzungsvlies wird typischerweise die Probe aufgebracht. Im Anschluss an das Freisetzungsvlies befindet sich eine poröse Membran, auf der die Bildung des Sandwich-Komplexes (konjugierter Antikörper-Antigen-Capture-Antikörper-Komplex) stattfindet. Am Ende einer Membran ist ein weiteres Vlies analog zum Saugvlies befestigt, das die aufgetragenen Flüssigkeiten sammelt und somit den kontinuierlichen, kapillaren Flüssigkeitsstrom über den gesamten Teststreifen hinweg gewährleistet.

Das Auftragen des mit kolloidalem Gold konjugierten Antikörpers SWLA1, des Antigens zum Dotieren (vorliegend ganze Zellen von S. mutans), des Capture-Antikörpers SWLA1 sowie der Antikörper auf der Kontrolllinie erfolgt wie oben erwähnt im Dispensierverfahren.

### 1.2 Gewinnung von Speichel

Speichelproben aus verschiedenen Patienten wurden erhalten, indem zunächst die Speichelbildung durch Kauen eines Paraffinpellets induziert wurde. Pro Testperson wurde ein Pellet verwendet. Der Speichel wurde in einem Meßbecher mit einem Gesamtvolumen von 30 ml aufgefangen. Der während der ersten 30 Sekunden gesammelte Speichel wurde verworfen, und der während der folgenden 5 Minuten gebildete Speichel wurde gesammelt und anschließend für die weitere Untersuchung verwendet.

### 1.3 Konditionierung von Speichel

Die Flussrate des gesammelten Speichels auf dem Teststreifen kann durch einen geeigneten Chromatographiepuffer vereinheitlicht werden. Typischerweise erreicht der Puffer innerhalb von einer Minute das Sammelvlies. Die Antigen-Antikörper-Reaktion bzw. die Ausbildung des Sandwich-Komplexes findet aber in der ganzen Phase des Chromatographieprozesses statt, d.h. solange noch Flüssigkeit vom Saug- zum Sammelvlies laufen kann. Die maximale Signalintensität entwickelt sich innerhalb der ersten 30 Minuten nach Chromatographiestart und bleibt dann für mindestens weitere 30 Minuten stabil.

Um eine bessere Zugänglichkeit der in der Probe vorhandenen Bakterien der Mundflora zu erreichen und auf diese Weise das Testsignal zu verstärken, können verschiedene Zusätze in den Laufpuffer gegeben werden. Zu diesem Zweck wurden Untersuchungen zur Speichelkonditionierung durchgeführt, bei denen jeweils 200 µl Speichel in 1.5 ml Reaktionsgefäßen mit 20 µl Testlösung durch viermaliges Umdrehen vorsichtig gemischt und 5 min bei Raumtemperatur inkubiert wurden, um so die Testbedingungen auf den Teststreifen bezüglich Inkubationszeit und Temperatur zu simulieren. Anschließend wurde eine Chromatographie auf Teststreifen durchgeführt und die Intensität des Testsignals sowie die Geschwindigkeit der Chromatographie beurteilt.

Es wurden verschiedene der oben beschriebenen Substanzen im Hinblick auf ihre Fähigkeit untersucht, die Intensität des Signals zu verbessern. Es konnte gezeigt werden, dass im Falle von Markierungen mit kolloidalem Gold insbesondere chaotrope Reagenzien und die Ionenstärke des Puffers erhöhende Reagenzien wie Kaliumiodid (0,001 - 2,0 M) und Natriumsulfat (0,001 - 2,5 M) sowie Lysozym (0,001 - 20 g/L), Papain (0,001 - 50 g/L) und Glutathion (0,001 - 100 g/L) die Intensität der Testsignale nachweisbar erhöhen konnten. Der Fachmann wird auf Basis der vorliegenden Beschreibung in der Lage sein, entsprechende Optimierungen für jedes auf einen bestimmten Mikroorganismus ausgerichtete Testsystem vorzunehmen.

### 1.4 Auftragen der Proben auf die Testvorrichtung

15 µl der gewonnenen Speichelproben wurden mittels einer Pipette auf den erfindungsgemäßen Teststreifen aufgebracht. Aufgrund der hohen Viskosität von Speichel wird die chromatographische Trennung durch Zugabe eines Laufmittels gestartet. Als Laufmittel dient vorliegend ein Puffer der folgenden Zusammensetzung: 20 mM TRIS, 0.5 % Rinderserumalbumin, 0.5 % Saccharose, 0.7 % Natriumdodecylsulfat, 0.5 % Tween 80, 1 % Natriumsulfat, 0.3 % Natriumcholat, 0.095 % Natriumazid, 0.05 % ProClin 300. Der pH-Wert wird mit Salzsäure auf einen Wert von 9,0 eingestellt. Der als Laufmittel dienende Puffer wird in ein Reservoir der Testvorrichtung überführt. Anschließend werden die aus der Testvorrichtung ragenden Enden der Teststreifen für mindestens 15 s (maximal 120 s) in Puffer getaucht, wodurch die Chromatographie gestartet wird.

### 1.5 Auswertung der Signalintensität

Innerhalb von 60 min, vorzugsweise von 5-30 min kann das Resultat durch Vergleich mit einem Vergleichsstandard abgelesen werden. Als Vergleichsstandard dient vorliegend ein optischer Standard, der mit definierten Konzentrationen von S. mutans erhalten wurde.

## Patentansprüche

1. Teststreifen für die Bestimmung des Kariesrisikos durch semi-quantitativen Nachweis eines kariogenen Mikroorganismus ausgewählt aus der Gruppe der Mutans-Streptokokken und Lactobacilli in einer flüssigen Speichel- oder Plaque-Probe, umfassend
a) einen ersten Bereich zur Aufnahme der flüssigen Speichel- oder Plaque-Probe, wobei der erste Bereich einen markierten Antikörper aufweist, der mit dem nachzuweisenden Mikroorganismus unter Ausbildung eines Immunkomplexes reagieren kann;
b) einen zweiten Bereich, der von dem ersten Bereich beabstandet ist und einen Capture-Antikörper aufweist, der mit dem nachzuweisenden Mikroorganismus unter Ausbildung eines Immunkomplexes reagieren kann, und auf dem Teststreifen immobilisiert ist;
wobei der Teststreifen derart gestaltet ist, dass sich der markierte Antikörper bei Kontakt des ersten Bereichs des Teststreifens mit einem Laufmittel in den zweiten Bereich des Teststreifens bewegt, sodass bei Vorliegen des nachzuweisenden Mikroorganismus in der flüssigen Speichel- oder Plaque-Probe, die im ersten Bereich aufgetragen wird, ein Immunkomplex aus dem markierten Antikörper, dem Capture-Antikörper und dem Mikroorganismus gebildet wird, der anhand der Markierung des markierten Antikörpers in dem zweiten Bereich des Teststreifens nachgewiesen werden kann, wobei die Stärke des von der Markierung bereitgestellten Signals Aufschluß über die Konzentration des Mikroorganismus in der flüssigen Speichel- oder Plaque-Probe gibt, und wobei der Teststreifen ferner eine Kontrolllinie umfasst, deren Intensität auf eine Konzentration von 10⁵ CFU des Mikroorganismus pro ml Speichelprobe eingestellt ist, was bei einem Erwachsenen einem mittleren Risiko entspricht, an Karies zu erkranken, sodass ein stärkeres Signal als die Kontrolllinie auf ein erhöhtes Risiko und ein schwächeres Signal als die Kontrolllinie auf ein niedriges Risiko verweist.

2. Teststreifen nach Anspruch 1, bei dem der markierte Antikörper und/oder der Capture-Antikörper ein monoklonaler Antikörper ist.

3. Teststreifen nach Anspruch 1 oder 2, bei dem der markierte Antikörper an Partikel konjugiert ist.

4. Teststreifen nach Anspruch 3, bei dem die Partikel aus kolloidalem Gold oder kolloidalem Silber bestehen.

5. Teststreifen nach Anspruch 1, bei dem der kariogene Mikroorganismus ausgewählt ist aus der Gruppe bestehend aus Streptococcus mutans, Streptococcus sobrinus, Lactobacillus acidophilus, Lactobacillus gasseri, Lactobacillus casei und Lactobacillus rhamnosus.

6. Kit, **dadurch gekennzeichnet, dass** es mindestens einen Teststreifen nach einem der Ansprüche 1-5 umfaßt.

7. Kit nach Anspruch 6, das ferner Puffer, Markierungsreagenzien und/oder Detektionsreagenzien zum Nachweis des markierten Antikörpers umfaßt.

## Claims

1. Test strip for the determination of the caries risk by semi-quantitative detection of a cariogenic microorganism selected from the group of mutans Streptococci and Lactobacilli in a liquid saliva or plaque sample, comprising:
a) a first area for accepting the liquid sample, said first area comprising a labelled antibody which is capable of reacting with the microorganism to be detected by forming an immunocomplex;
b) a second area spaced from the first area, comprising a capture antibody which is capable of reacting with the microorganism to be detected by forming an immunocomplex and which is immobilised on the test strip;
wherein the test strip is designed such that the labelled antibody, upon contact of the first area of the test strip with a mobile phase moves into the second area of the test strip such that in the presence of the microorganism to be detected in the liquid saliva or plaque sample which is applied in the first area, an immunocomplex is formed from the labelled antibody, the capture antibody and the microorganism, which complex can be detected by means of the labelling of the labelled antibody in the second area of the test strip, wherein the intensity of the signal provided by the labelling provides information on the concentration of the microorganism in the liquid saliva or plaque sample, and wherein the test strip further comprises a control line, whose intensity is adjusted to a concentration of 10⁵ CFU of the microorganism per ml saliva sample, which in the case of an adult corresponds to a medium risk of developing caries, so that a stronger signal than the control line indicates an increased risk and a weaker signal than the control line indicates a lower risk.

2. Test strip according to claim 1, wherein the labelled antibody and/or the capture antibody is a monoclonal antibody.

3. Test strip according to claim 1 or 2, wherein the labelled antibody is conjugated to particles.

4. Test strip according to claim 3, wherein particles consist of colloidal gold or colloidal silver.

5. Test strip according to claim 1, wherein the cariogenic microorganism is selected from the group consisting of Streptococcus mutans, Streptococcus sobrinus, Lactobacillus acidophilus, Lactobacillus gasseri, Lactobacillus casei, and Lactobacillus rhamnosus.

6. Kit, **characterized in that** it comprises at least one test strip according to any of claims 1-5.

7. Kit according to claim 6, which further comprises buffers, labelling reagents and/or detection reagents for detecting the labelled antibody.

## Revendications

1. Bande de test pour la détermination du risque de carie, par détection semi-quantitative d'un micro-organisme cariogène choisi dans le groupe des streptocoques mutants et des lactobacilles, dans un échantillon liquide de salive ou de plaque dentaire, comprenant :
a) une première partie destinée à recevoir l'échantillon liquide de salive ou de plaque dentaire, la première partie présentant un anticorps marqué qui peut réagir avec le micro-organisme à détecter, en formant un complexe immun;
b) une deuxième partie qui est espacée de la première partie et présente un anticorps de capture qui peut réagir avec le micro-organisme à détecter, en formant un complexe immun, et est immobilisé sur la bande de test;
la bande de test étant configurée de manière telle que lors du contact de la première partie de la bande de test avec une phase mobile, l'anticorps marqué se déplace dans la deuxième partie de la bande de test, de sorte qu'en présence du micro-organisme à détecter dans l'échantillon liquide de salive ou de plaque dentaire qui est appliqué dans la première partie, un complexe immun est formé à partir de l'anticorps marqué, de l'anticorps de capture et du micro-organisme, complexe qui peut être détecté dans la deuxième partie de la bande de test à l'aide du marquage de l'anticorps marqué, l'intensité du signal fourni par le marquage donnant des renseignements sur la concentration du micro-organisme dans l'échantillon liquide de salive ou de plaque dentaire, et la bande de test comportant en outre une ligne de contrôle dont l'intensité est réglée sur une concentration de 10⁵ CFU du micro-organisme par ml d'échantillon de salive, ce qui correspond pour un adulte à un risque moyen de développer des caries, de sorte qu'un signal plus fort que la ligne de contrôle indique un risque accru et un signal plus faible que la ligne de contrôle indique un risque réduit.

2. Bande de test selon la revendication 1, dans laquelle l'anticorps marqué et/ou l'anticorps de capture est un anticorps monoclonal.

3. Bande de test selon la revendication 1 ou 2, dans laquelle l'anticorps marqué est conjugué à des particules.

4. Bande de test selon la revendication 3, dans laquelle les particules sont constituées d'or colloïdal ou d'argent colloïdal.

5. Bande de test selon la revendication 1, dans laquelle le micro-organisme cariogène est choisi dans le groupe comprenant streptococcus mutans, streptococcus sobrinus, lactobacillus acidophilus, lactobacillus gasseri, lactobacillus casei et lactobacillus rhamnosus.

6. Kit, **caractérisé en ce qu'**il comprend au moins une bande de test selon une des revendications 1 à 5.

7. Kit selon la revendication 6, comprenant en outre des tampons, des réactifs de marquage et/ou des réactifs de détection pour déceler la présence de l'anticorps marqué.
